# EUROPEAN PATENT APPLICATION

(11) **EP 2 840 518 A2**
(43) Date of publication of application: **25.02.2015**
(21) Application number: 14180437.7
(22) Date of filing: 08.08.2014
(51) Int. Cl.: G06F 19/00

(54) **Managing dental photographs**

(30) Priority: 12.08.2013 US 201313964981
(71) Applicant: Dental Imaging Technologies Corporation, Hatfield, PA 19440 (US)
(72) Inventor: Cocco, George John, Havertown, PA 19083 (US); Parma, Michael Joseph, Chalfont, PA 18914 (US)
(74) Representative: Garratt, Peter Douglas

(57) **Abstract**

Methods, systems, and computer-readable medium containing instructions for managing patient image information. One method includes displaying within a graphical user interface a list of dental patients on a portable computing device. The method also includes receiving a selection of a patient from the list from a user input and capturing at least one optical, extraoral photograph of the selected patient. The method further includes associating, on the portable computing device, the at least one optical, extraoral photograph with additional information including information identifying the selected patient, and transmitting the at least one optical, extraoral photograph and the additional information to a computer physically separate from the portable computing device for storage in an electronic record associated with the selected patient.

## Description

### FIELD

Embodiments of the invention relate to medical imaging systems, for example, dental imaging systems. In particular, embodiments of the invention relate to systems and methods for managing dental images acquired using optical imaging devices including, for example, camera-equipped smart phones, tablet computers, and intelligent digital cameras.

### BACKGROUND

Most dental imaging software products allow photographs to be imported into an image library from a digital camera. For example, a dentist may take standard photographs of a patient using a digital camera. To upload the acquired images to the dentist's image library, the dentist must connect the camera to a host computer through a connection (for example, a universal serial bus ("USB") connection) or by mounting the camera media as a separate drive on the host computer. After the images are uploaded, the dentist must manually add patient and other information to the images before the images are linked to existing patient files stored in the image library on or accessible via the computer.

### SUMMARY

Conventional methods for adding photographs to a patient's record require multiple manual steps, which are burdensome and prone to human error. Therefore, in some situations, the dentist may not take the time to actually upload the photographs, which makes the photographs unusable by others and not securely saved with the patient's electronic record. Furthermore, because the photographs are stored on the dentist's camera untitled or unmatched to a particular patient, the dentist may incorrectly remember or completely forget what photographs are associated with what patients. In addition, because the dentist can use a handheld digital camera to capture a photograph of a patient from myriad distances, angles, and positions, the resulting photographs are often inconsistent, which make them less usable for making comparisons with other photographs of the same patient or across multiple patients.

Therefore, embodiments of the invention provide methods and systems for allowing a user to select patient information prior to taking a photograph of a particular patient. The captured photograph is then uploaded (in some instances automatically) to the user's image library or patient record database or store matched to or associated with the selected patient without requiring later manual data entry of patient information. In some embodiments, the user can also select additional information before taking the photograph. This additional information can include image type and a timeline tag indicating during what stage of treatment the image was taken (e.g., "before," "progress," or "after"). These user-selections are associated with the captured images and uploaded with the image to the image library, which allows a user to search and identify photographs based on the type of photograph or a particular timeline tag. Also, in some embodiments, a user can select a photo guide for taking a photograph, which helps the user take consistent photographs. The guide used to take a particular photograph can also be associated with the uploaded photograph, which allows a user to search and identify photographs taken using a particular photo guide.

For example, one embodiment provides a method for managing patient information. The method includes displaying within a graphical user interface a list of dental patients on a portable computing device. The method also includes receiving a selection of a patient from the list from a user and capturing at least one optical, extraoral photograph of the selected patient. The method further includes associating, on the portable computing device, the at least one optical, extraoral photograph with additional information including information identifying the selected patient, and transmitting the at least one optical, extraoral photograph and the additional information to a computer physically separate from the portable computing device for storage in an electronic record associated with the selected patient. The record can contain clinical information about the patient, including, for example, 2D x-ray images, 3D x-ray images, diagnoses, treatment plans, and/or intraoral or extraoral photographs.

Another embodiment provides a system for managing patient information. The system includes a portable computing device including a digital camera and a controller. The controller is configured to display a list of dental patients on the portable computing device within a graphical user interface, receive a selection of a patient from the list from a user, and to capture at least one optical, extraoral photograph of an object using the digital camera. The controller is also configured to associate the at least one optical, extraoral photograph with additional information including information identifying the selected patient, and to transmit the at least one optical, extraoral photograph and the additional information to a computer physically separate from the portable computing device for storage in an electronic record associated with the selected patient and containing patient clinical information. In certain circumstances it is useful to take multiple photographs of a patient and in such cases multiple photographs are captured and transmitted.

Yet another embodiment of the invention provides a non-transitory computer readable medium containing instructions for managing patient information. The medium includes instructions for displaying a list of dental patients on a portable computing device within a graphical user interface, receiving a selection of a patient from the list from a user, and capturing at least one optical, extraoral photograph of the selected patient. The medium also includes instructions for associating the at least one optical, extraoral photograph with additional information including information identifying the selected patient, and transmitting the at least one optical, extraoral photograph and the additional information to a computer physically separate from the portable computing device for storage in an electronic record associated with the selected patient and containing patient clinical information.

Other aspects of the invention will become apparent by consideration of the detailed description and accompanying drawings.

### BRIEF DESCRIPTION OF THE DRAWINGS

FIG. 1 illustrates a dental imaging system.
FIG. 2 schematically illustrates a portable computing device included in the system of FIG. 1.
FIG. 3 is a flow chart illustrating a method of managing patient information performed by the portable computing device of FIG. 2.
FIGS. 4-14 and 16 are drawings illustrating a graphical user interface displayed on the portable computing device of FIG. 2.
FIG. 15 is a drawing illustrating a graphical user interface displayed on a viewing workstation included in the system of FIG. 1.

### DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

Also, it is to be understood that the phraseology and terminology used herein is for the purpose of description and should not be regarded as limiting. The use of "including," "comprising" or "having" and variations thereof herein is meant to encompass the items listed thereafter and equivalents thereof as well as additional items. The terms "mounted," "connected" and "coupled" are used broadly and encompass both direct and indirect mounting, connecting and coupling. Further, "connected" and "coupled" are not restricted to physical or mechanical connections or couplings, and can include electrical connections or couplings, whether direct or indirect. Also, electronic communications and notifications may be performed using other known means including direct connections, wireless connections, etc.

It should also be noted that a plurality of hardware and software based devices, as well as a plurality of different structural components may be utilized to implement the invention. Furthermore, and as described in subsequent paragraphs, the specific configurations illustrated in the drawings are intended to exemplify embodiments of the invention and that other alternative configurations are possible.

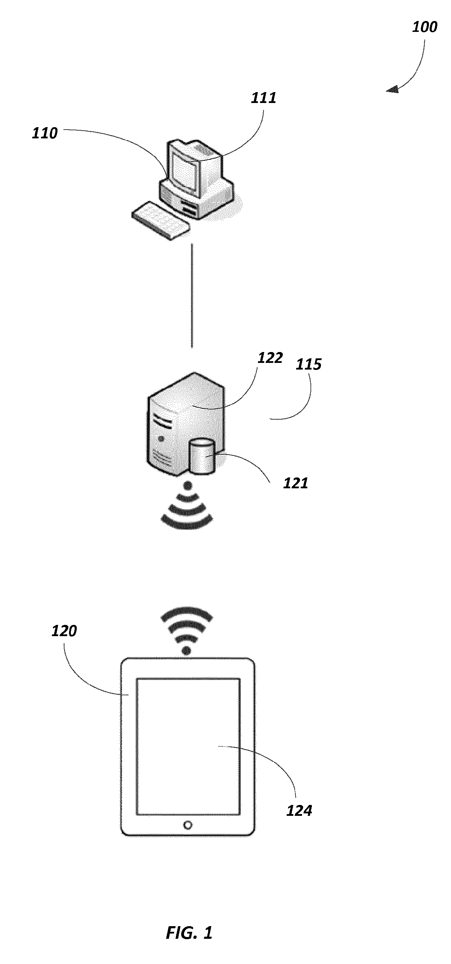

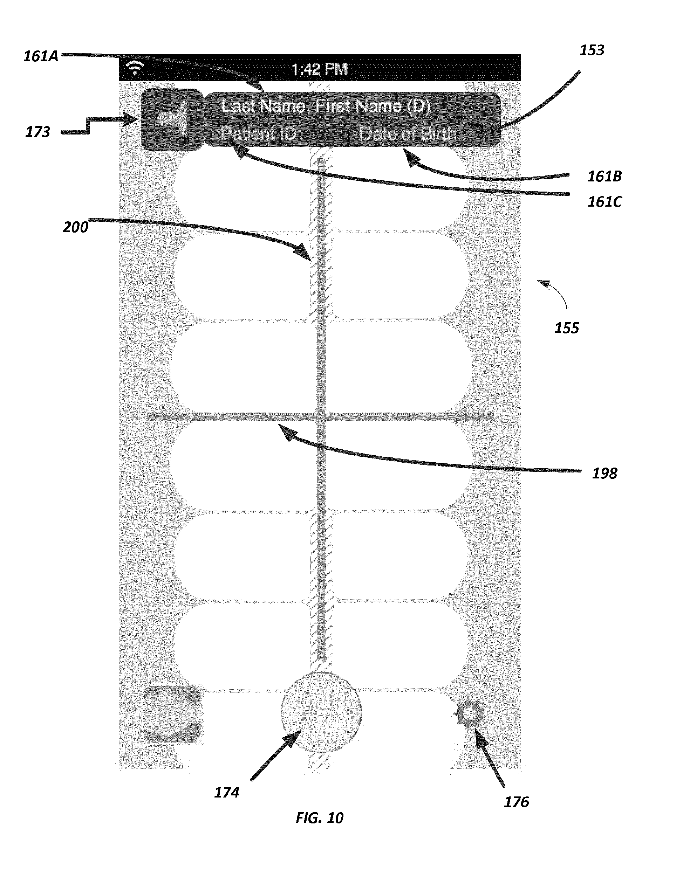

FIG. 1 illustrates a dental imaging system 100. The system 100 includes a viewing workstation 110 having a display 111, a storage server 115, and a portable computing device 120. The viewing workstation 110 (hereinafter referred to as the "workstation") includes standard computing components, for example, a processing unit (e.g., a microprocessor), memory modules storing non-transitory data and instructions executable by the processing unit, and an input/output interface for communicating with external devices and systems. For example, the workstation 110 stores executable instructions for accessing images and associated information stored in the storage server 115 and displaying a graphical user interface containing the images and associated information on a monitor connected to the workstation 110 (hereinafter referred to as the "viewing software"). In some embodiments, the viewing software also allows a user to add or modify images and/or the associated information and store the additions and modifications to the storage server 115.

The storage server 115 (hereinafter referred to as the "server") includes an image library 121. The image library 121 stores images and associated information. The associated information can include a patient identifier, automatically-generated metadata (e.g., time, date, geographic location, device identifier, etc.) and, optionally, user-selected additional information. Instead of or in addition to the image library 121, the storage server 115 can include an electronic patient record database 122. Each record in the database 122 is associated with a patient and contains patient information including, for example, the patient's name, date of birth, and clinical information. The clinical information can include, for example, medical history, medications being taken by the patient, diagnoses, treatments, clinical images, etc. Clinical or non-clinical images stored in or otherwise associated with a patient record can include images captured as described herein. In some embodiments, the server 115 includes a Microsoft Standard Query Language ("SQL") server that allows files (e.g., images and associated information) to be shared among devices and provides remote or web-based services (e.g., remote image access). The server 115 can include similar computing components as described above for the workstation 110 (i.e., a processing unit, memory modules, and an input/output interface). The server 115 also stores instructions executable by the processing unit to manage the image library 121 and/or the electronic patient record database 122 (hereinafter referred to as the "server software"). The server software can respond to requests and information transmitted from the workstation 110 (i.e., from the viewing software).

For example, as illustrated in FIG. 1, the server 115 is connected to and communicates with the workstation 110 using a wired connection, a wireless connection, or a combination thereof. In some embodiments, the server 115 communicates with the workstation 110 over a local area network using various communication protocols. The communication protocols include, for example, common internet file system ("CIFS"), SQL, and transmission control protocol ("TCP"). The server 115 also communicates with the portable computing device 120. The portable computing device 120 can be, for example, a smart phone, tablet computer or other handheld computing device. Optionally, the portable computing device 120 may be an iPhone, iPad, or iPod Touch sold by Apple Inc.

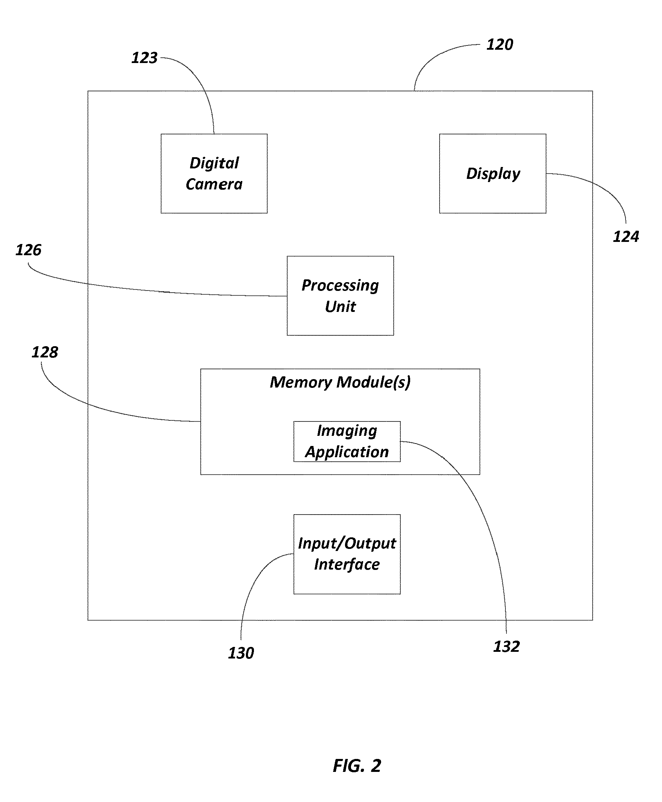

As illustrated in FIG. 2, the portable computing device 120 includes a digital camera 123, a display 124, a processing unit 126 (e.g., a microprocessor), one or more memory modules 128, and an input/output interface 130. Although not illustrated in FIG. 2, one or more of the components of the portable computing device 120 are connected by one or more connections or communication paths either directly and/or using a communication bus. It should also be understood that the portable computing device 120 can also include additional components than those illustrated in FIG. 2.

In the embodiment shown, the digital camera 123 is built into the device 120 and includes at least one lens for capturing an optical, extraoral photograph. An extraoral photograph is broadly any photographic image showing the outside of the mouth and/or face and can include clinical images focused on a patient's jaw and teeth as well as an identification image. (Herein, an extraoral photograph is sometimes simply referred to as an "image".) In some embodiments, the digital camera 123 includes a first lens on the front of the portable computing device 120 and a second lens on the back or rear of the device. A device with two such lenses is capable of capturing an image of a subject on either side of the portable computing device 120. In some embodiments, the digital camera 123 also includes a flash. The display 124 can include a liquid crystal display ("LCD") screen and, in some embodiments, is a touchscreen. As an alternative or in addition to the touchscreen, the portable computing device 120 can include a user interface that includes one or more tactile buttons or other selection mechanisms. These selection mechanisms may also be referred to as user inputs.

The memory modules 128 includes non-transitory computer-readable medium. The non-transitory computer-readable medium includes, for example, random-access memory ("RAM") and/or read-only memory ("ROM"). The processing unit 126 retrieves instructions from the memory modules 128 and executes the instructions to perform particular functionality. The processing unit 126 can also retrieve and store data to the memory modules 128 as part of executing the instructions. For example, as illustrated in FIG. 2, the memory modules 128 store an imaging application 132. The imaging application 132 includes instructions and data executable by the processing unit 126 to manage images captured using the digital camera 123. In particular, as described in more detail below, the imaging application 132 automatically associates user-selected information including, for example, a patient identifier, with images captured using the digital camera 123 and automatically transmits the images and the associated information to the storage server 115.

The processing unit 126 can also exchange data with external devices and systems through the input/output interface 130. For example, as illustrated in FIG. 2, the portable computing device 120 uses the input/output interface 130 to wirelessly communicate with the server 115. For example, the portable computing device 120 can wirelessly communicate with the server 115 using the hypertext transport protocol ("HTTP") and/or the hypertext transport protocol secure ("HTTPS"). The portable computing device 120 can communicate with the server 115 over a local area network (e.g., the workstation 110, server 115, and portable computing device 120 can be connected over a local area network) or over a wide area network including, for example, the Internet.

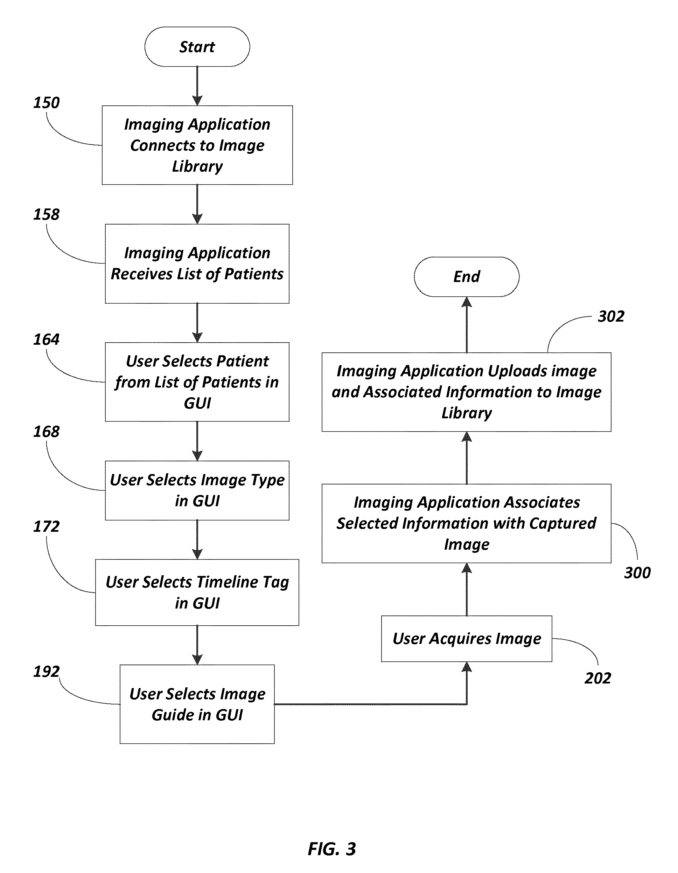

As noted above, the imaging application 132 includes instructions that when executed by the processing unit 126 capture images, associate the images with user-selected information, and automatically upload images with the associated information to the server 115. For example, FIG. 3 illustrates a method of managing dental patient information performed by the portable computing device 120 using the imaging application 132. As illustrated in FIG. 3, the imaging application 132 connects with dental image library 121 or patient record database 122 stored on the server 115 (at block 150). The imaging application 132 retrieves from the server 115 a list of dental patients 153. In some embodiments, the list of patients 153 can be tailored for the user. For example, based on an identifier input into the imaging application 132 by the user, the imaging application 132 can retrieve only those patients associated with the identified user. Also, in some embodiments, the imaging application 132 is configured to retrieve only a subset of available patients from the server 115. For example, the application 132 can be configured to select only a predetermined number of the most recent patients. The user may also be able to set the parameters for retrieving patients based on settings for the application 132. In addition, in some embodiments, the imaging application 132 is configured to retrieve patients from the server 115 based on a query entered by the user. Also, in some embodiments, the list of patients 153 is based on patients open or recently accessed on the workstation 110. For example, the server 115 can be configured to communicate with the workstation 110 to identify one or more patient files open or recently accessed on the workstation 110 through the viewing software. Alternatively or in addition, the server 115 can store a log of patient files recently accessed.

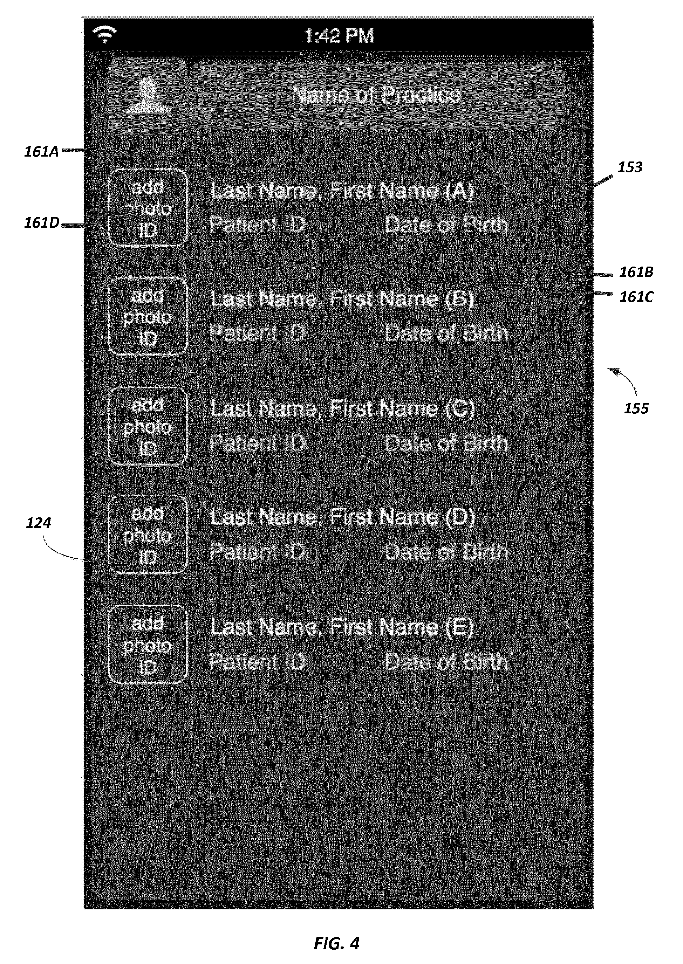

In response to receiving the list of patients 153 or patient data from the server 115, the imaging application 132 generates a graphical user interface ("GUI") 155 that includes the list of patients 153 and outputs the GUI 155 to the display 124 (at block 158). For example, FIG. 4 illustrates a display 124 including the GUI 155 and a list of patients 153. In some embodiments, as illustrated in FIG. 4, the list of patients 153 includes a patient name 161A, a patient birthdate 161B, a unique patient identifier 161C, and an identification picture 161D, if such a picture exists for the patient. From the list of patients 153, the user can select one of the patients to capture an image of the selected patient (at block 164).

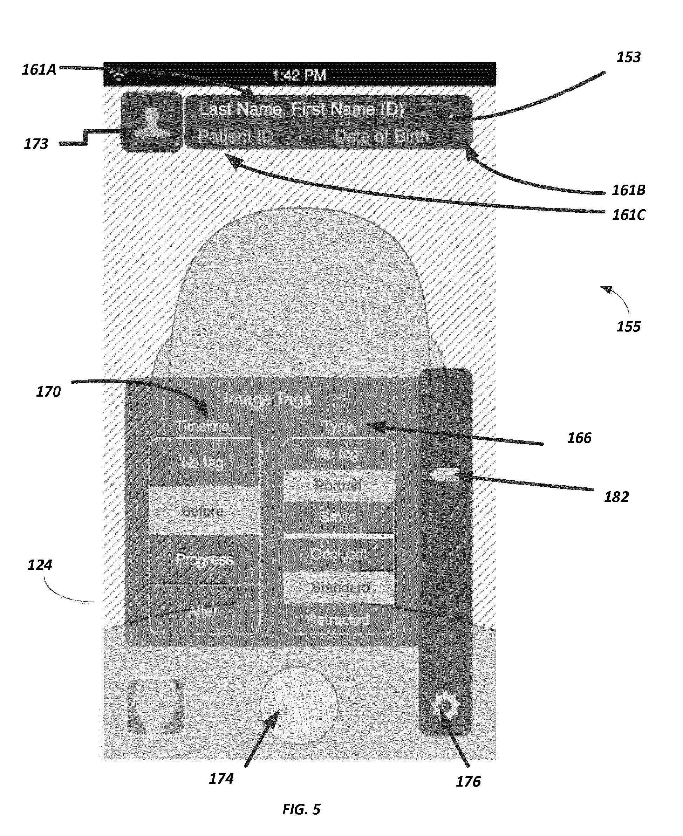

Optionally, upon selecting a patient, the imaging application 132 uses the GUI 155 to prompt the user for additional information associated with the selected patient and the image that the user wants to acquire of the selected patient, as shown in FIG. 5. The additional information can include an image type 166. For example, using the GUI 155, the user can select a type of image 166 he or she wants to take of the selected patient (at block 168). In the embodiment shown, the image type 166 can include "no tag," "portrait," "smile," "occlusal," "standard," "retracted." Other image types are possible, for example, "patient ID photo." The additional information can also include a timeline tag 170. In particular, using the GUI 155, the user can select a timeline designation for the image that he or she wants to take (at block 172). The GUI 155 can be configured to provide a list of available timeline designations. The timeline tags 170 define a particular position or time or within a sequence spanning a course of treatment. In the illustrated embodiment, the timeline tags 170 include "before," "progress," "after," or "no tag." In other embodiments, the GUI 155 allows the user to define a customized timeline tag 170. The timeline tags 170 can also include a phase, treatment, or session number (e.g., "session 1," "session 2," etc.). The illustrated GUI also includes a navigation button 173 that returns the user to the patient list illustrated in FIG. 4.

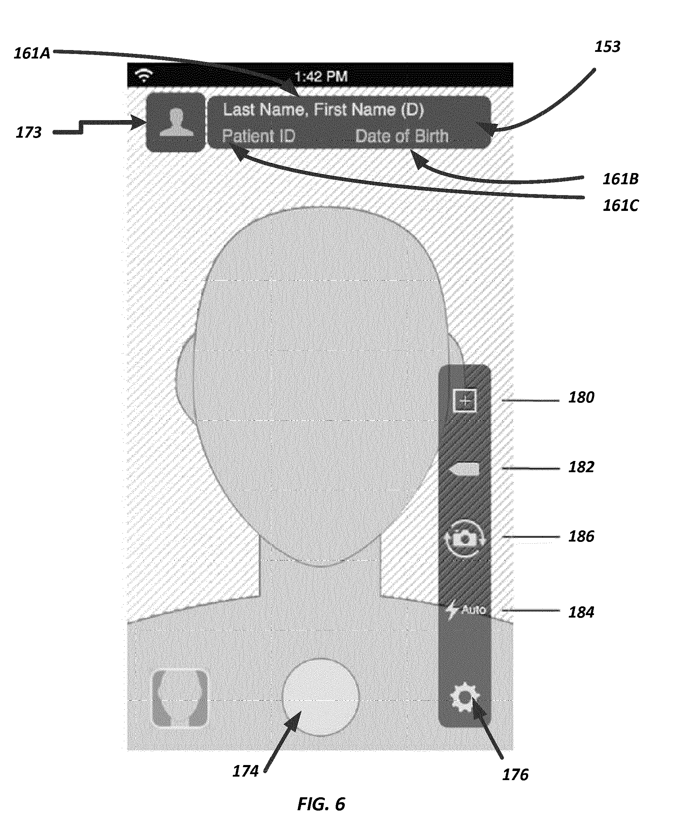

In the illustrated embodiment, the GUI 155 also provides an image capturing selector 174, which captures the image of the patient, and a settings selector 176 that allows a user to set or adjust various image capture parameters, as shown in FIG. 6. For example, as illustrated in FIG. 6, the settings selector 176 includes an image guide selector 180, image tag selector 182, which allows the user to assign an image type 166 and/or a timeline tag 170 as previously described, a flash selector 184 that allows a user to turn the flash on, turn the flash off, or set the flash to automatic, and a camera lens selector 186, which allows the user to select a front or back lens if the portable computing device 120 includes more than one lens. Other image capture parameters can include zooming, contrast adjustment, brightness adjustment, color or black and white selectors, etc.

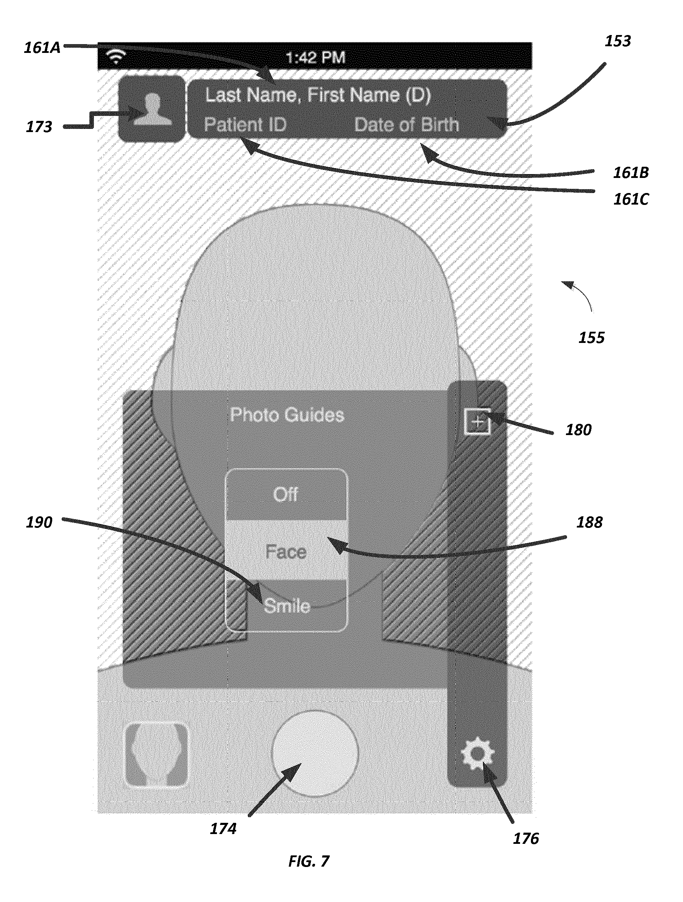

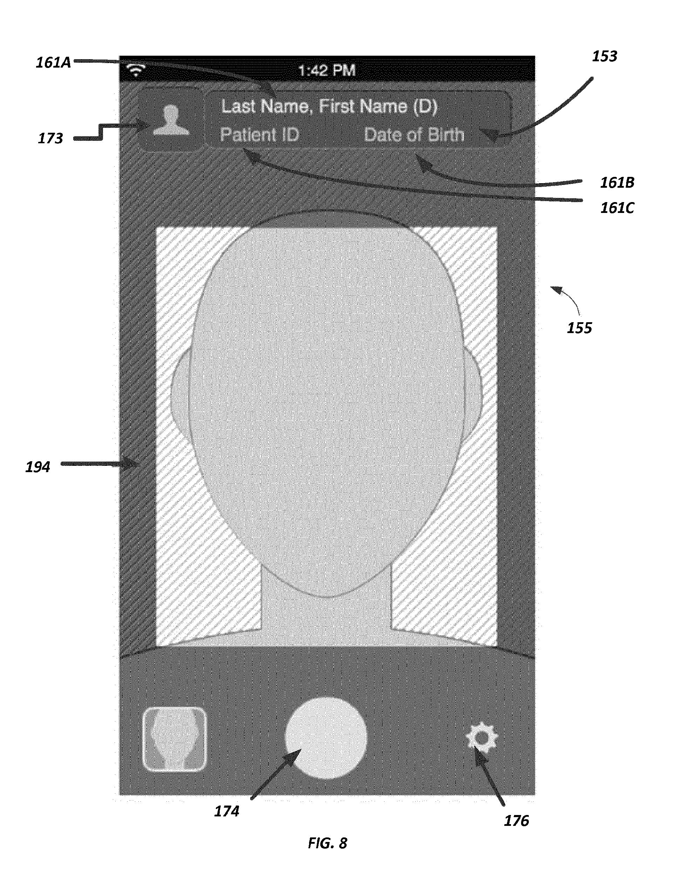

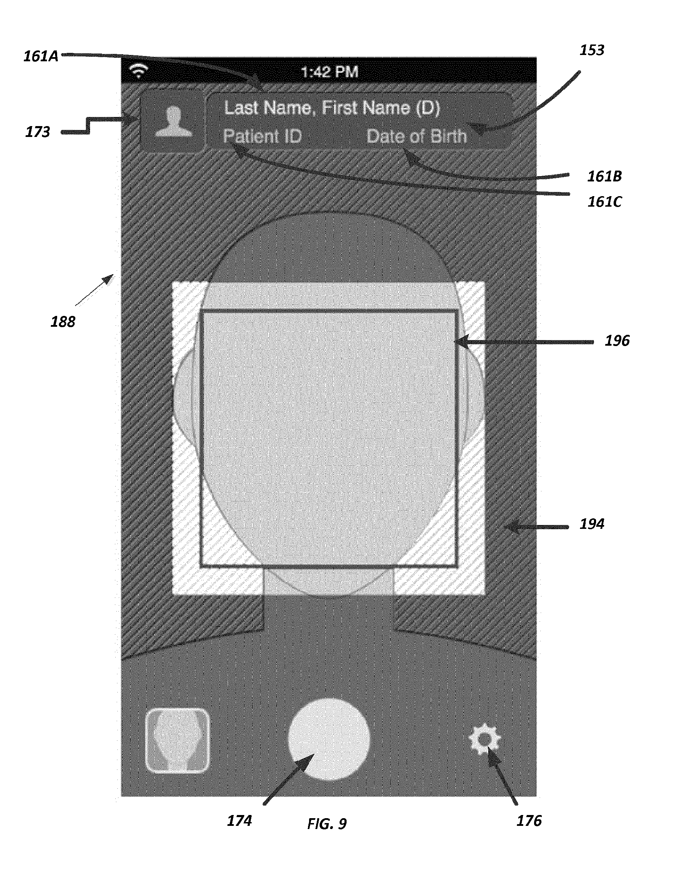

In the illustrated embodiment, the imaging application 132 generates an image guide ("Photo Guide") selector 180, as illustrated in FIG. 7. In the illustrated embodiment, a user may turn the photo guide feature "off" or select a portrait guide "face" 188 or a "smile" photo guide 190. Use of a photo guide helps in accurately and consistently capturing an image. For example, if the user wants to take a particular type of image, the user can select an image guide in the GUI 155 (at block 192). If the user wants to take a portrait image, the user can select a portrait guide 188. The portrait guide 188 provides guides (e.g., lines or contours) for aligning with the patient's head and/or shoulders. The portrait guide 188 includes a partially opaque template 194, as shown in FIG. 8, that overlays the live camera display on the portable computing device 120. In some embodiments, the portable computing device 120 includes a face detection feature 196 as illustrated in FIG. 9. The face detection feature 196 includes a box that defines the perimeter of what the portable computing device 120 considers the face. To properly align a subject, the face detection feature 196 box and the opaque template 194 are aligned. The user can use the portrait guide 188 to ensure that he or she captures the entire portrait of the subject and that all portrait images taken using the portable computing device 120 are consistent. Similarly, if the user wants to take a smile image, the user can select a smile guide 190. The smile guide 190, as illustrated in FIG. 9, provides a centerline 198 and bite plane 200 guide lines for centering the patient's mouth within the acquired image. In some embodiments, the image guide 160 also provides feedback to the user regarding whether the user has properly aligned a subject with the guide. For example, guide lines displayed as part of the guide can flash or change color to indicate when the user has properly aligned the subject.

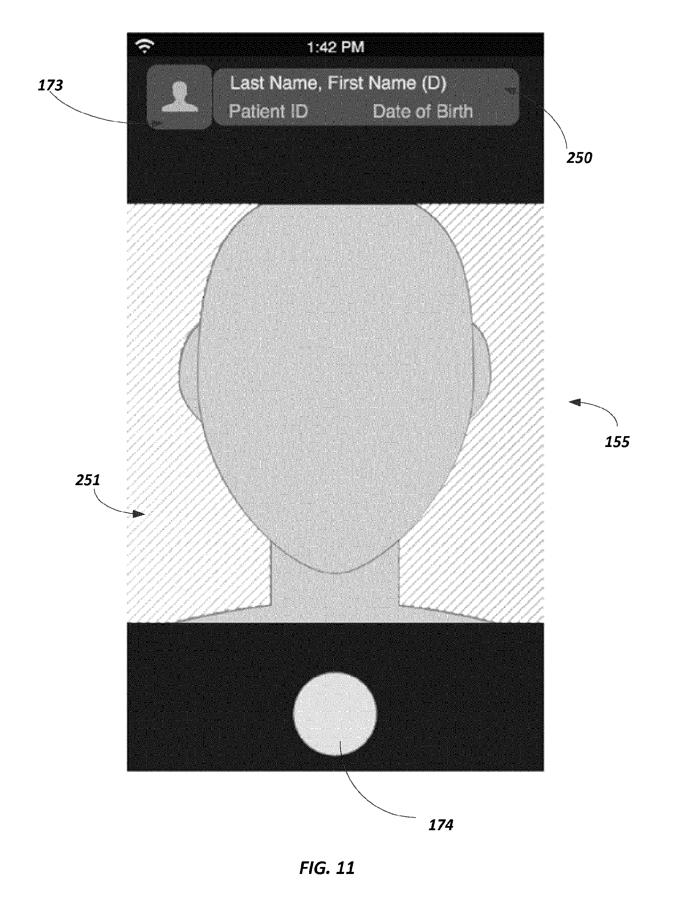

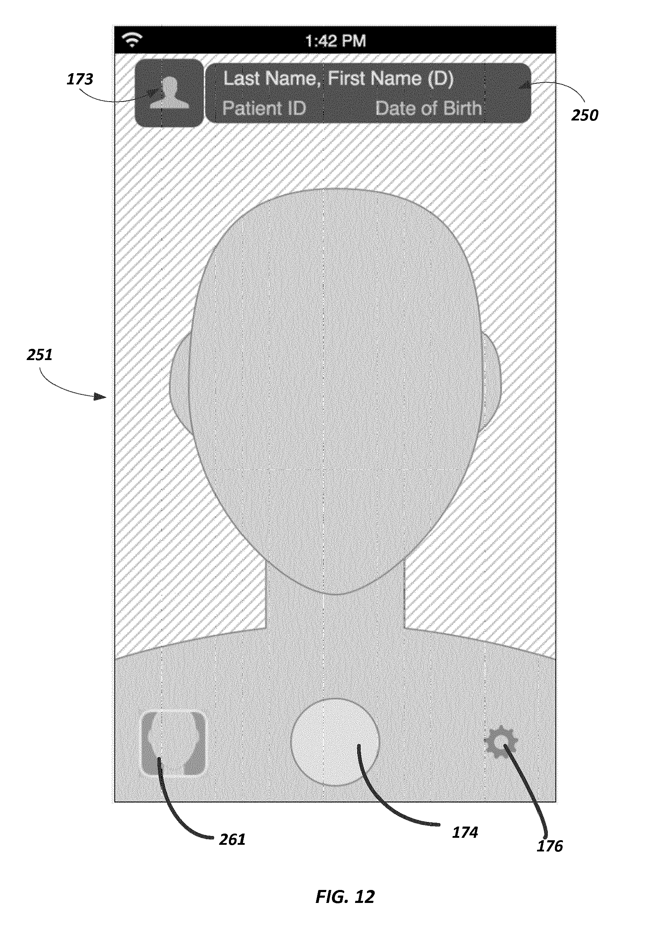

After the user has selected all of the information to be associated with the image and set the camera parameters through the GUI 155(i.e., the patient identifier and, optionally, the additional information), the user can use the digital camera 123 included in the portable computing device 120 to acquire a picture of a subject (i.e., the selected patient) (at block 202). In some embodiments, the portable computing device 120 executes the imaging application 132 to capture an image (e.g., through the GUI 155 provided by the application 132). In other embodiments, the portable computing device 120 executes a separate image capture application. For example, FIG. 11 illustrates the GUI 155 generated by the imaging application 132 used to capture an image. As illustrated in FIG. 11, the GUI 155 identifies the selected patient in a banner 250 of the GUI 155 (labeled with the fictitious name "Last Name, First Name (D)" in FIG. 11) with a live camera display 251. The user selects the image capturing selector 174 to capture the image displayed within the live camera display 251 of the GUI 155. As illustrated in FIG. 11, in some embodiments, the live camera display 251 is cropped within the GUI 155 (e.g., a square crop). In other embodiments, as illustrated in FIG. 12, the live camera display 251 spans a majority of the GUI 155.

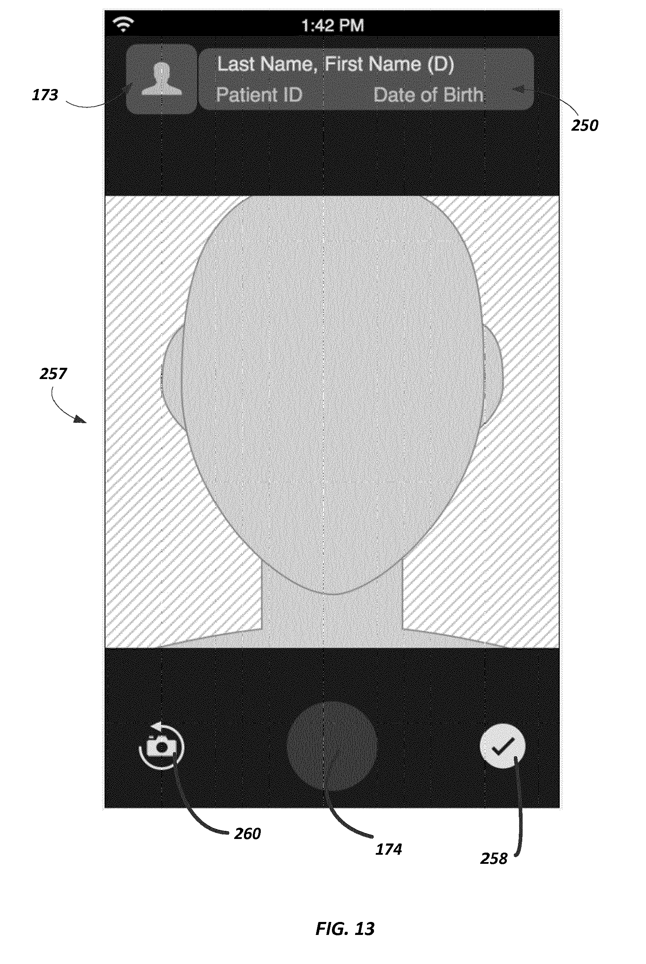

After the user captures an image, the GUI 155 can display the captured image 257 as illustrated in FIG. 13. The GUI 155 can also include an accept selector 258, which allows the user to accept and save the captured image, and a retake selector 260, which allows the user to retake the image. In some embodiments, if the user selects the retake selector 260, the imaging application 132 deletes the captured image 257 and allows the user to retake an image using the GUI 155 illustrated in FIGS. 11 and 12. In other embodiments, if the user selects the retake selector 260, the imaging application 132 retains previously-captured images. For example, as illustrated in FIG. 12, the GUI 155 can include a review selector 261, which allows a user to view one or more previously-captured images. In some embodiments, the GUI 155 generated by the imaging application 132 also provide image editing tools. Image editing tools include, for example, a crop tool, a zoom tool, a brightness adjust tool, a rotate tool, etc.

After the user takes (and, optionally, accepts) an image, the imaging application 132 automatically associates the image with the user selected information, including selected patient and any selected additional information with the captured image (at block 300). In some embodiments, the imaging application 132 also automatically associates the image with metadata. Metadata includes, for example, a time, a date, a geographic location, a device identifier, etc. The imaging application 132 then uploads or transmits the captured image and the associated information to the server 115 (at block 302). In some embodiments, the imaging application 132 automatically performs the upload after the user takes the image. In other embodiments, the imaging application 132 waits for user instruction to perform the upload (e.g., waits until the user accepts a captured image or until the user clicks "Upload").

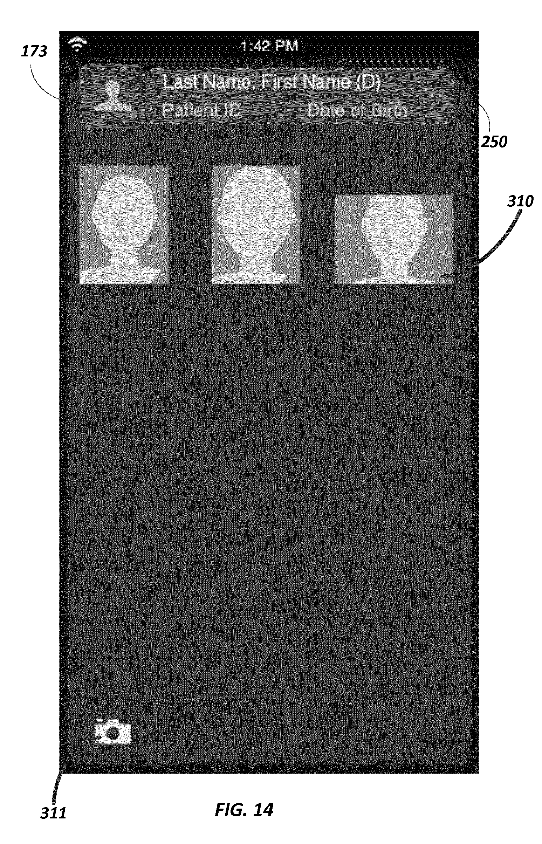

The imaging application 132 can also inform a user whether a particular image (and the associated information) was uploaded. For example, as illustrated in FIG. 14, the imaging application 132 can display a list of previously-captured images, a status indicator 310, for example, the triangle icon in the bottom right corner of each image, and a new picture selector 311, which allows the user to take another image of the patient. The status indication 310 indicates whether an image (and the associated information) was successfully transferred to the server 115. Uploaded images can be saved on the portable computing device 132 until the user closes the imaging application 132 or selects a new patient. In other embodiments, the imaging application 132 automatically deletes a captured image once the image is successfully uploaded to the server 115. If a network or other failure occurs during transfer of an image (and the associated information) to the server 115, the imaging application 132 can save the image until the application 132 can re-attempt the upload.

Accordingly, when the image is uploaded to the image library 121 and the electronic patient record database 122 stored on the server 115, the image is pre-matched with a particular patient record and preferably does not require any manual matching of the image with a particular patient post-production of the image. Therefore, images captured by a user using the portable computing device 120 are quickly and easily added to the image library 121 and the electronic patient record database 122, where they are usable as being associated with a particular patient. Based on the additional information associated with the uploaded image, the user can also better manage and categorize uploaded images (e.g., using the viewing software executed by the workstation 110). For example, the user can query the image library 121 for those images associated with a particular image type 166, a particular timeline tag 170, and/or a particular image guide 188, 190. In particular, the user can use this additional information stored with the uploaded images to quickly find "before" and "after" pictures for all patients, a "before" picture for a particular patient, all "portrait" images for all patients, all "retracted" images for a particular patient, all images taken using the "smile" photo guide, and so on.

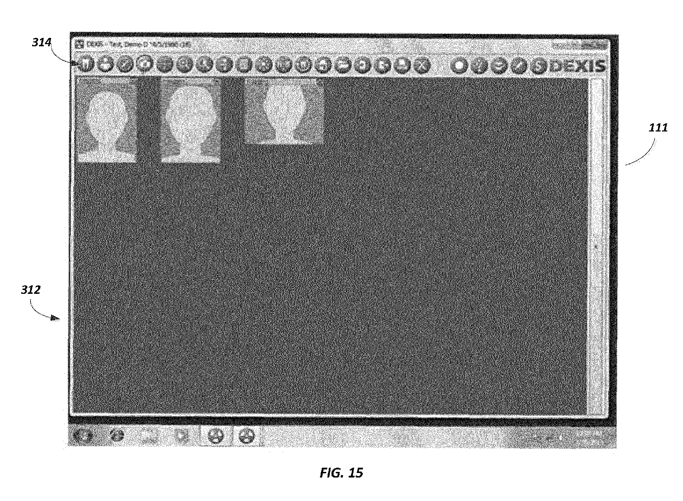

For example, FIG. 15 illustrates a GUI 312 provided by the workstation 110 on the display 111 that a user can use to view and manage uploaded images. In the illustrated embodiment, the GUI 312 displays images from the electronic patient record database 122 or the image library 121. The GUI also includes several other selectors 314 to modify the images, the information associated with the images, the information associated with the patient. The selectors 314 may also include actions to be performed with the electronic patient record or the image(s), for example, printing, e-mailing, referring a patient to an outside provider, etc.

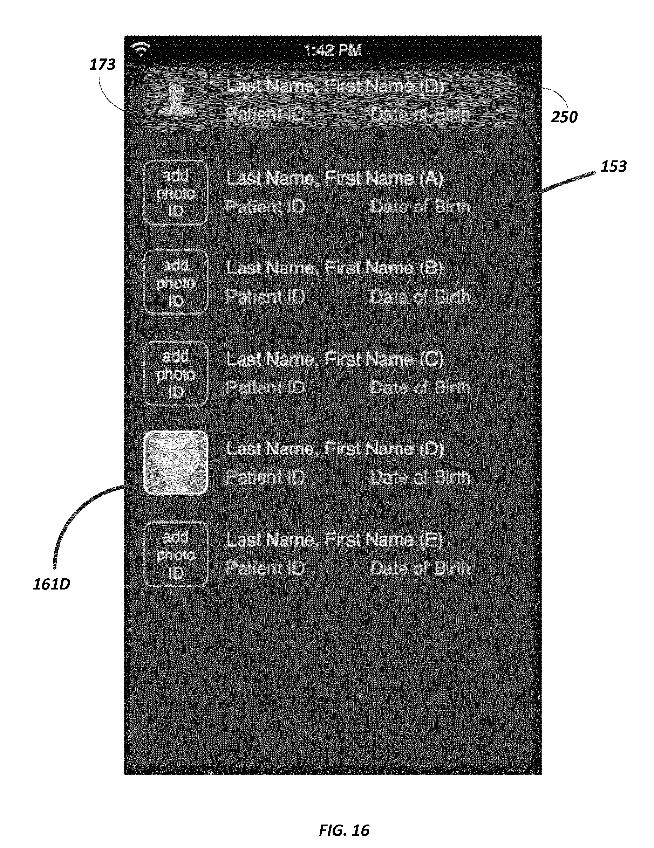

It should be understood that the imaging application 132 can be used to upload other types of image to the image library 121. For example, as noted above, a user can use the imaging application 132 to capture and upload an identification picture 161D for the patient. The identification picture 161D can be associated with a particular patient record and, in some embodiments, is displayed as part of the list of patients 153, as illustrated in FIG. 16. Furthermore, it should be understood that the imaging application 132 can be used as described above to upload video images to the image library 121 or the electronic patient record database 122. Furthermore, in some embodiments, the imaging application 132 can be used to upload other data items to the server 115. For example, any data item commonly stored or collected using a portable computing device can be associated with a particular patient and uploaded to the server 115. Accordingly, a user can use the imaging application 132 to upload voicemails or emails received from particular patients on the user's portable computing device 120. It should also be understood that although embodiments of the invention have been described with respect to dental patients and dental-related images, the imaging application 132 can be used for other types of subjects and other types of industries including medical imaging applications.

Various features and advantages of the invention are set forth in the following claims.

## Claims

1. A method of managing patient information comprising:
displaying within a graphical user interface a list of dental patients on a portable computing device;
receiving, by the portable computing device, a user input indicating a selected patient from the list;
capturing at least one optical, extraoral photograph of the selected patient;
associating, on the portable computing device, the at least one optical, extraoral photograph with additional information, the additional information including information identifying the selected patient;
transmitting the at least one optical, extraoral photograph and the additional information from the portable computing device to a computer physically separate from the portable computing device; and
storing, by the computer, the at least one optical, extraoral photograph and the additional information in an electronic record associated with the selected patient, the electronic record containing clinical information related to the selected patient.

2. The method according to claim 1, wherein the step of capturing at least one optical, extraoral photograph of the selected patient comprises capturing at least two optical, extraoral photographs of the selected patient.

3. The method according to any one of the preceding claims, wherein the portable computing device comprises a built-in camera, and wherein the step of capturing the at least one optical, extraoral photograph comprises capturing the at least one optical, extraoral photograph with the built-in camera.

4. The method according to any one of the preceding claims, wherein the additional information further comprises at least one of an image type and a timeline tag.

5. The method according to claim 4, wherein the image type indicates at least one of a portrait, a smile image, an occlusal image, a standard image, a patient identification picture, and a retracted image.

6. The method according to claim 4, wherein the timeline tag indicates at least one of a time before treatment, progress during treatment, and a time after treatment.

7. The method according to any one of the preceding claims, wherein the capturing step comprises presenting an image guide to a user.

8. The method according to any one of the preceding claims, further comprising receiving, by the portable computing device, the list of dental patients from the computer.

9. The method according to any one of the preceding claims, wherein the transmitting step comprises automatically transmitting the at least one optical, extraoral photograph and the additional information to the computer after the associating step.

10. The method according to any one of the preceding claims, further comprising receiving an accept selection from the user for the at least one optical, extraoral photograph, and wherein the transmitting step comprises automatically transmitting the at least one optical, extraoral photograph and the additional information in response to receiving the accept selection.

11. The method according to any one of the preceding claims, wherein the transmitting step comprises transmitting the at least one optical, extraoral photograph and the additional information wirelessly.

12. Apparatus for managing patient information comprising:
a portable computing device including a digital camera and a controller; and
a computer physically separate from the portable computing device, wherein the portable computing device is configured to:
display on the portable computing device a list of dental patients within a graphical user interface,
receive a user input indicating a selected patient from the list,
capture at least one optical, extraoral photograph of an object using the digital camera,
associate the at least one optical, extraoral photograph with additional information, the additional information including information identifying the selected patient, and
transmit the at least one optical, extraoral photograph and the additional information to the computer, and wherein the computer is configured to store the at least one optical, extraoral photograph and the additional information in an electronic record associated with the selected patient, the electronic record containing clinical information related to the selected patient.

13. The apparatus according to claim 12, wherein the portable computing device comprises a handheld computing device.

14. The apparatus according to claim 12 or claim 13, wherein the controller is configured to transmit the at least one optical, extraoral photograph and the additional information through a wireless link.

15. The apparatus according to any one of claim 12 to claim 14, wherein the additional information further comprises at least one of an image type and a timeline tag.

16. The apparatus according to claim 15, wherein the image type indicates at least one of a portrait, a smile, an occlusal image, a standard image, a patient identification picture, and a retracted image.

17. The apparatus according to claim 15, wherein the timeline tag includes at least one of a time before treatment, progress during treatment, and a time after treatment.

18. The apparatus according to any one of claim 12 to claim 17, wherein the portable computing device is further configured to present an image guide to a user.

19. The apparatus according to any one of claim 12 to claim 18, wherein the image guide includes a partially opaque overlay for a live camera display that includes at least one guideline for aligning a portion of a subject in the live camera display.

20. The apparatus according to any one of claim 12 to claim 19, wherein the controller is configured to transmit the at least one optical, extraoral photograph and the additional information automatically after associating the additional information with the at least one optical, extraoral photograph.

21. The apparatus according to any one of claim 12 to claim 20, wherein the controller is further configured to receive an accept selection from the user and is configured to transmit the at least one optical, extraoral photograph and the additional information in response to receiving the accept selection.

22. At least one non-transitory computer readable medium containing instructions for managing patient information, the at least one medium comprising instructions for:
displaying, by a portable computing device, a list of dental patients within a graphical user interface;
receiving, by the portable computing device, a user input indicating a selected patient from the list;
capturing at least one optical, extraoral photograph;
associating, on the portable computing device, the at least one optical, extraoral photograph with additional information, the additional information including information identifying the selected patient;
transmitting the at least one optical, extraoral photograph and the additional information from the portable computing device to a computer physically separate from the portable computing device; and
storing, by the computer, the at least one optical, extraoral photograph and the additional information in an electronic record associated with the selected patient, the electronic record containing clinical information related to the selected patient.
